# EUROPEAN PATENT APPLICATION

(11) **EP 0 774 512 A2**
(43) Date of publication of application: **21.05.1997**
(21) Application number: 96306713.7
(22) Date of filing: 16.09.1996
(51) Int. Cl.: C12N 15/31, C12N 15/70, C12N 1/21, C07K 1/113, C07K 14/195, C07K 14/32, C12P 21/02

(54) **A method for production of protein using molecular chaperon**

(30) Priority: 14.09.1995 JP 237176/95; 29.08.1996 JP 228252/96
(71) Applicant: Imanaka, Tadayuki, Suita-shi, Osaka (JP)
(72) Inventor: Imanaka, Tadayuki, Suita-shi, Osaka (JP); Takagi, Masahiro, Suita-shi, Osaka (JP); Fujiwara, Shinsuke, Nishinomiya-shi, Hyogo-ken (JP); Kohda, Katsunori, Suita-shi, Osaka (JP); Kubomi, Tomoko, Higashiosaka-shi, Osaka (JP); Yan, Zhen, c/o The Centre of Biotechnology, Xian 710032 (CN)
(74) Representative: W.P. Thompson & Co.

(57) **Abstract**

An expression cassette which can express a soluble form of a desired protein in a bacterial cell, wherein the cassette comprises a sequence in which a gene encoding a molecular chaperon is operably linked to a first promoter and a site to which a gene encoding the desired protein can be inserted is provided. Also, a method for expressing a desired protein in a soluble form is provided by the use of the expression cassette or co-transformation with a plasmid which can express a molecular chaperon and a plasmid which can express the desired protein.

## Description

The present invention relates to a method for efficiently producing a desired protein. More specifically, the present invention relates to a method for producing a protein in a bacterial cell as a soluble protein. More precisely, the present invention is related to manufacture of a protein expressed in a bacterial cell as a soluble, active protein that is normally expressed in a bacterial cell as an insoluble, inactive protein; an expression cassette or an expression vector; and a transformant used for the method.

A cell is not always found under ideal conditions. The cell is exposed to various stresses such as changes in temperature, pH, etc. It is known that when a cell is exposed to a high temperature, the cell produces a group of specific proteins known as "heat shock proteins" (HSP) (Ellis, R.J. et., al (1990) Molecular Chaperons: The plant connection. Science 250: 954-959). The HSP described in this publication is known as a molecular chaperon and is constitutively expressed. The role of the molecular chaperon has been carefully studied and it has been found to be involved in biological functions common among different species, such as formation and maintenance of the higher structure of the protein, membrane permeation of a protein, regulation of cell cycle, origin and differentiation of a cell, and functions of the immunological system. (Zeilstra-Ryalls, J., O. Fayet and C.Georgo. (1991) The universally conserved GroE(Hsp60) chaperonins. Annu. Rev. Microbiol. 45: 301-325, Ellis, R.J. et., al. (1991) Molecular Chaperons. Annu. Rev. Biochem. 60: pp. 321-347). HSPs are classified into the following 5 families by their molecular weights:

| | |
|---|---|
| 1. HSP60 (chaperonin) family | GroEL, Hsp60, Cpn60 |
| 2. Hsp70 family | DnaK, Hsp70, Bip |
| 3. Hsp90 family | HtpG, Hsp90, Grop94 |
| 4. TRiC family | TF55, TRiC (TCP1) |
| 5. other family | GroES, Hsp28, Hsp45 |

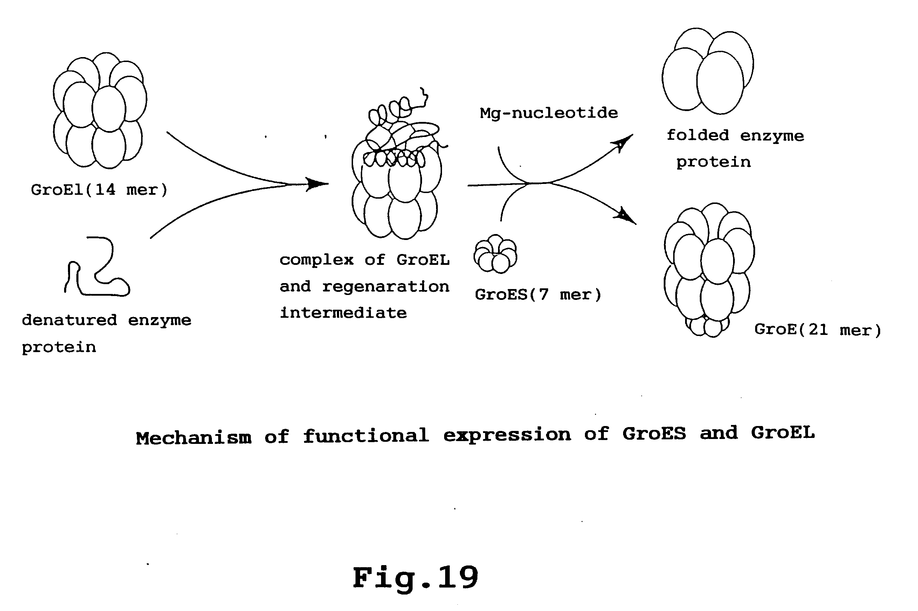

Recently, a HSP was found to aid in formation of conformation and higher structure of a protein even in vitro. Therefore, the elucidation of the structure and function of such a HSP becomes important. A HSP which is involved in the conformation and the conformational change of a protein is GroEL. GroEL, when combined with GroES having a molecular weight of a subunit 10KDa, has been shown to aid higher structure formation of various proteins in vivo or in vitro. GroEL has ATPase activity and has a characteristic 14 mer quaternary structure composed of two 7 mer doughnut shaped subunits. GroES, like the subunits of GroEL, is considered to be a 7 mer and has a doughnut-like structure. The 14 mer of GroEL and the 7 mer of GroES form a complex at a 1:1 ratio in vivo and acts as a GroE protein (figure 19: Yasushi Kamata BIO VIEW (1993)). Among chaperonin proteins, GroE has been well studied with respect to their involvement in the formation of protein structure. In the eukaryotic cell, a protein called "t-complex polypeptide-1" (TCP1) has been found to activate ATP dependent actin formation or tubulin formation in vitro. (Gao et., al. (1992) A cytoplasmic chaperonin that catalyzes β-actin folding. Cell 69: pp.1043-1050; and Yaffe et., al. (1992) TCP1 complex is a molecular chaperon tubulin biogenesis. Nature 358: pp.245-248). Recently, it has been reported that hyperthemophilic archaeon has a TCP-1-like molecular chaperon (TF55) (Jonathern D. et., al. (1991) A molecular chaperon from hyperthermophilic archaebacterium is related to the eukaryotic protein t-complex polypeptide-1. Nature 354: pp.490-493).

In thermophilic bacteria, all the biopolymers are stabilized in order to tolerate the high temperature. Therefore, proteins derived from thermophilic bacteria are applied to various fields such as polymerase chain reaction and biosensor (Saiki et., al. (1988) Primer-directed enzymatic amplification of DNA with a thermostable DNA polymerase.Science 239: 487-491; and Kagawa et., al. (1989) Biotechnological applications of thermophilic ATP synthetase. Membrane electronics and genetics. J. Membrane Sci. 41: pp.237-247). Since molecular chaperon from thermophilic and hyperthermophilic archaebacterium are considered to have high stability, they are extremely useful for studying mechanisms of higher conformational structure formation, artificially induced formation of a higher structure of a desired protein, or renaturation.

In the field of genetic engineering, in order to produce a desired protein in a large amount and for efficient recovery, a bacterial cell is generally used as a host since the bacterial cell is easy to grow and to manipulate. However, in a bacterial cell, a foreign protein is mostly expressed in an insoluble and inactive form such as an inclusion body. Also, in the case where a foreign promoter which can function in a bacterial cell is used, the protein expressed is an insoluble, inactive protein.

The recovered insoluble, inactive protein can then be treated to solubilize and reactivate it. In the case where the insoluble protein is a heat stable enzyme, a heat treatment is conducted to solubilize the insoluble protein. However, since recovery is low, a method for expressing a protein in soluble form is required.

In one aspect of the present invention there is provided an expression cassette which can express a desired protein in a host cell, wherein the cassette comprises a sequence in which a gene encoding a molecular chaperon is operably linked to a first promoter and a site to which a gene encoding the desired protein can be inserted.

In one embodiment of the present invention, the expression cassette is functional in a bacterial cell.

In one embodiment of the present invention, the expression cassette can express a protein in a soluble form, which is expressed as an insoluble form in a bacterial cell in the absence of the molecular chaperon.

In one embodiment of the present invention, the expression cassette has a second promoter, and the second promoter is present upstream of the insertion site and is located so as to promote expression of the inserted gene.

In another embodiment of the present invention, the expression cassette has a terminator sequence downstream of the gene encoding the molecular chaperon and downstream of the site to which the gene encoding the desired protein is inserted.

In still another embodiment, the gene encoding the desired protein is inserted as an expressible form.

In still another embodiment, the gene encoding the molecular chaperon is a heat shock protein (HSP) gene of a hyperthermophilic archaeon KOD-1.

In still another embodiment, the gene encoding the molecular chaperon is a GroESL gene of Bacillus stearothermophilus SICI.

In still another embodiment, both the first and the second promoter are T7 promoters.

In another aspect of the present invention there is provided an expression vector comprising the above expression cassette, the desired gene being operably incorporated into the cloning site.

The present invention further relates to a cell which can express a desired protein, wherein the cell is transformed with an expression cassette or an expression vector containing the expression cassette, and wherein the cassette comprises a sequence in which a gene encoding a molecular chaperon is operably linked to a first promoter and a site to which a gene encoding the desired protein can be inserted.

In another aspect of the present invention there is provided a cell which can express a desired protein in a soluble form, wherein the bacterial cell is co-transformed with a vector which can express a gene encoding a molecular chaperon and a vector which can express a sequence encoding the desired protein.

In one embodiment of the present invention, the expression cassette is functional in a bacterial cell.

In one embodiment of the present invention, the expression cassette can express a protein in a soluble form, which is expressed as an insoluble form in a bacterial cell in the absence of the molecular chaperon.

In one embodiment of the present invention, the gene encoding the molecular chaperon is a heat shock protein gene of a hyperthermophilic archaeon KOD-1.

In another embodiment of the present invention, the gene encoding the molecular chaperon is a GroESL gene of Bacillus stearothermophilus SICI.

In yet another aspect of the present invention there is provided a method for expressing a desired protein in a soluble form, wherein the method comprises a step of culturing a cell which can co-express a gene encoding a molecular chaperon and a gene encoding the desired protein.

In one embodiment of the present invention, the cell is transformed with a vector having a gene encoding a molecular chaperon being operably linked to a first promoter and having a gene encoding the desired protein operably linked to a second promoter.

In another embodiment of the present invention, the cell is co-transformed with a vector which can express a gene encoding a molecular chaperon and a vector which can express a sequence encoding the desired protein.

In still another embodiment of the present invention, the gene encoding the molecular chaperon is a heat shock protein gene of a hyperthermophilic archaeon KOD-1.

In still another embodiment of the present invention, the gene encoding the molecular chaperon is a GroESL gene of Bacillus stearothermophilus SICI.

In one embodiment of the present invention, the host cell is a bacterial cell.

In one embodiment of the present invention, the desired protein is expressed in a soluble form, which is expressed as an insoluble form in the absence of the molecular chaperon.

In a further aspect of the present invention there is provided a method for expressing a desired protein in a soluble form comprising:
culturing a cell having an expression cassette or an expression vector containing a gene encoding a molecular chaperon and a gene encoding the desired protein and co-expressing the molecular chaperon and the desired protein;
heating the cell culture broth or a fraction containing the desired protein;
separating an insoluble fraction; and
recovering the desired protein.

In one embodiment of the present invention, the cell is transformed with a vector having a gene encoding a molecular chaperon being operably linked to a first promoter and having a gene encoding the desired protein operably linked to a second promoter.

In another embodiment of the present invention, the cell is co-transformed with a vector which can express a gene encoding a molecular chaperon and a vector which can express a sequence encoding the desired protein.

In still another embodiment of the present invention, the gene encoding the molecular chaperon is a heat shock protein gene of a hyperthermophilic archaeon KOD-1.

In still another embodiment of the present invention, the gene encoding the molecular chaperon is a GroESL gene of Bacillus stearothermophilus SICI.

In one embodiment of the present invention, the host cell is a bacterial cell.

In one embodiment of the present invention, the desired protein is expressed in a soluble form, which is expressed as an insoluble form in the absence of the molecular chaperon.

In a further aspect of the present invention there is provided a method for changing a heat liable protein to heat stable protein comprising mixing the heat liable protein and a heat stable molecular chaperon.

In a further aspect of the present invention there is provided a method for purifying a heat liable protein comprising:
mixing the heat liable protein and a heat stable molecular chaperon; and heating the mixture.

In a further aspect of the present invention there is provided a heat shock protein of KOD-1 comprising an amino acid sequence of SEQ ID NO: 7.

In a further aspect of the present invention, there is provided a gene encoding a heat shock protein of KOD-1 comprising an amino acid sequence of SEQ ID NO: 7.

Thus, the invention described herein makes possible the advantages of providing:
a protein which is expressed in a cell, for example a bacterial cell, as an insoluble inclusion body that can be expressed in the bacterial cell as a soluble protein, by using vector(s) which can express the molecular chaperon and the desired protein simultaneously, thereby making it possible to recover the desired protein efficiently.

These and other advantages of the present invention will become apparent to those skilled in the art upon reading and understanding the following detailed description with reference to the accompanying figures.

Figure 1 shows cloning of a GroESL gene.

Figure 2 is a diagram for preparing a plasmid pET-GroESL.

Figure 3 shows an expression cassette of the present invention.

Figure 4 shows construction of a plasmid pET-sFV.

Figure 5 is a continuation of Figure 4.

Figure 6 shows construction of an expression vector pET-sFV-ESL.

Figure 7 shows that the sFV is solubilized when a molecular chaperon is expressed simultaneously.

Figure 8 shows a restriction map of an EcoRI-HindIII fragment of hyperthermophilic archaebacterium KOD-1 and binding portions of probes having the sequence of sequence ID No.5 and 6.

Figure 9 shows a sequence of a HSP gene of hyperthermophilic archaebacterium KOD-1 and deduced amino acid sequence (546 amino acids).

Figure 10 is a SDS-PAGE of a protein expressed by a transformant of a plasmid pACYC-KOD Hsp.

Figure 11 shows a gel filtration pattern of the dimer form (120KDa) and polymer form (about 950KDa : 16mer) of HSPs.

Figure 12 shows the in vitro heat stability of ADH.

Figure 13 shows the heat stability of ADH when HSP is present.

Figure 14 shows the in vitro heat stability of ADH at 50°C.

Figure 15 shows a co-transformation using the expression vector of the present invention.

Figure 16 shows an increase in production of neutral amylase co-expressed with HSP.

Figure 17 shows a solubilization of CobQ when CobQ is co-expressed with HSP.

Figure 18 shows a solubilization of sFv when sFv is co-expressed with HSP.

Figure 19 is a scheme for a formation of a functional protein GroE, which is a complex of GroEL and GroES.

As used herein, "cell" means a prokaryotic cell or eukaryotic cell and includes bacterial cells, yeast cells, plant cells and mammalian cells.

As used herein, "bacterial cell" means a prokaryotic cell and archaebacterium. As a prokaryotic cell, both gram positive and gram negative bacterial cells are included.

As used herein, "foreign protein" means a protein which is not naturally found in the host (bacterial) cell. "Foreign promoter" means a promoter which is not naturally found in the host (bacterial) cell or a promoter which is not a respective natural promoter for expressing a protein.

As used herein, "soluble" means that substantially no inclusion bodies are found under microscopic observation.

Herein after, examples are described with respect to bacterial cells. However, it will be readily apparent to those skilled in the art that the examples can be applied to yeast cells, plant cells and mammalian cells.

### (Expression cassette)

An expression cassette of the present invention comprises a sequence in which a gene encoding a molecular chaperon is operably linked to a first promoter and a site to which a gene encoding the desired protein can be inserted. This expression cassette can be made by using a promoter and a gene encoding a molecular chaperon. A terminator sequence can also be used if necessary. As a promoter, a bacterial promoter and a phage promoter can be used. Preferably, tac promoter, lac promoter, etc., can be used. Most preferably, T7 promoter can be used for reasons of high expression.

As a molecular chaperon, heat shock protein (HSP) of hyperthemophilic archaeon, and GroEL, GroES, Hsp90, SecB or others derived from thermophilic bacteria such as Bacillus stearothermophilus, can be used. Among the proteins, HSP of hyperthemophilic archaeon is preferably used. Archaea is considered to be a taxonomic group different from prokaryotes or eukaryotes. Interest in archaea, which includes hypersalt tolerant archaeon, methane producing archaeon and hyperthermophilic archaeon, concerns the evolutional aspects of the group. The HSP from hyperthemophilic archaeon is most preferably used since the HSP is composed of one molecule.

Thermophilic bacteria or thermophilic archaeon of the present invention refer to bacteria or archaeon which grow in temperatures exceeding 60°C.

The hyperthermophilic archaeon preferably used in the present invention is defined as growing in temperatures more than 80°C.

Among the Hyperthermophilic archaeon, KOD-1 is preferably used. KOD-1 is a thermophilic thiol protease producing strain which is isolated from a solfatara wharf on Kodakara Island, Kagoshima, Japan (Appl. Environ. Microbiol. 60(12), pp.4559-4566 (1994)). KOD-1 is deposited in the National Institute of Bioscience and Human-Technology Agency of Industrial Science and Technology as accession No. FERM P-15007. KOD-1 was at first classified into genus Pyrococcus. However, as described in the reference above, KOD-1 is now considered to be more closely related to genus Thermococcus than genus Pyrococcus according to the comparison of 16S rRNA sequences.

GroEL and GroES from thermophilic bacteria can be preferably used since GroEL and GroES are known to bind to a molten globule, which is an intermediate shape of a folded protein, and promote the folding of the protein. The amino acid sequence and the nucleotide sequence of the Escherichia coli (E.coli) GroEL and GroES are described in Nature vol.333, pp.330-334 (1988).

As a starting vector for construction of the cassette of the present invention, any vector which is stably maintained and replicated in the bacterial cell can be used for construction of the cassette of the present invention. As the starting vector, pBR322, pUC18, pUC119, pET-8c and so on can be used. Preferably, pET-8c which has a bactriophage T7 f10 promoter can be used.

An example for construction of the expression cassette will now be described:

A first promoter is introduced into the starting vector. Then, a gene sequence encoding the molecular chaperon is ligated downstream of the first promoter. Introduction and ligation of these sequences can be done by a method or technique which is known to those skilled in the art. In order to obtain optimum expression activity, the distance between the first promoter sequence and the gene of the molecular chaperon can be regulated. As a molecular chaperon sequence, a known sequence can be used. A HSP gene, which is a molecular chaperon of the hyperthermophilic archaeon KOD-1, can be cloned by use of the conserved amino acid sequence of the chaperonin gene of the known HSP gene. Details of the screening are described in the Example.

A terminator sequence can be positioned downstream of the molecular chaperon. A T7 phage terminator sequence can be preferably used. The terminator sequence is useful for enhancing an expression efficiency. The ligation of the gene of the molecular chaperon and the terminator sequence can be performed using a method known to those skilled in the art.

A plasmid having a promoter sequence-molecular chaperon gene-terminator sequence can be constructed by inserting the molecular chaperon gene in-between the promoter sequence and terminator sequence of a plasmid, such as pET-8c, having the promoter sequence and terminator sequence. The thus obtained molecular chaperon expression vector can be an expression cassette of the present invention if the vector has a suitable cloning site of a gene of a desired protein. If the constructed vector does not have a suitable cloning site, a cloning site can be made so as to construct the expression cassette of the present invention. As the cloning site, a multi-linker which has a various restriction sites can be used. The multi-linker can be purchased from a commercial source or chemically synthesized.

To the cloning site of the expression cassette, a gene encoding the desired protein to which a second promoter is operably linked can be introduced.

An expression vector having a second promoter sequence is introduced upstream of the cloning site. The introduced second promoter can be the same as or different from the first promoter. The length of the linker can be regulated so as to efficiently express the desired protein. A terminator sequence can be introduced downstream of the cloning site.

### (Expression vector)

The expression vector of the present invention means a vector to which a gene encoding a desired protein is incorporated and includes an expression cassette to which the cloning site of the gene encoding the desired protein is introduced.

### (Transformation)

A method for transforming the expression vector to a bacterial cell is well known to those skilled in the art. For example, when Escherichia coli is used as a host, CaCl₂ treatment is employed. Screening of the transformant is also well known to those skilled in the art. The transformant is selected by the use of drug resistance or auxotrophy, with drug resistance is being the generally used method. As the drug resistance gene, ampicillin gene, chloramphenicol gene, tetracycline gene and so on can be used.

The transformant of the present invention does not always have both a molecular chaperon gene and a desired protein gene in the same plasmid. The transformant of the present invention can be co-transformed with a vector having the first promoter and the molecular chaperon gene and a vector having the second promoter and the desired protein gene. The two vectors used for co-transformation preferably each have a different drug resistance gene for selection.

### (manufacture of a desired protein)

The selected transformant can be cultivated by a method known to those skilled in the art. After the cultivation, cells are destroyed by a known method such as sonication, treatment with lysozyme, and so on. After centrifugation, the desired protein can be purified and recovered by a method using, for example, ammonium sulfate, ion exchange chromatography, column chromatography or affinity chromatography or combination thereof.

Proteins, which are expressed as an inclusion body in the bacterial cell and can be used in the present application are, but not limited to, plant proteins, or animal proteins such as antibodies.

### Examples:

### (Example 1 : construction of expression vector)

As a molecular chaperon, GroESL of Bacillus stearothermophilus SICI (herein after referred to as SICI) was used. The SICI is obtained by culturing a Bacillus stearothermophilus SI1 which was deposited to National Institute Bioscience and Human-Technology Agency of Industrial Science and Technology as a deposition No. FERM P-9629.

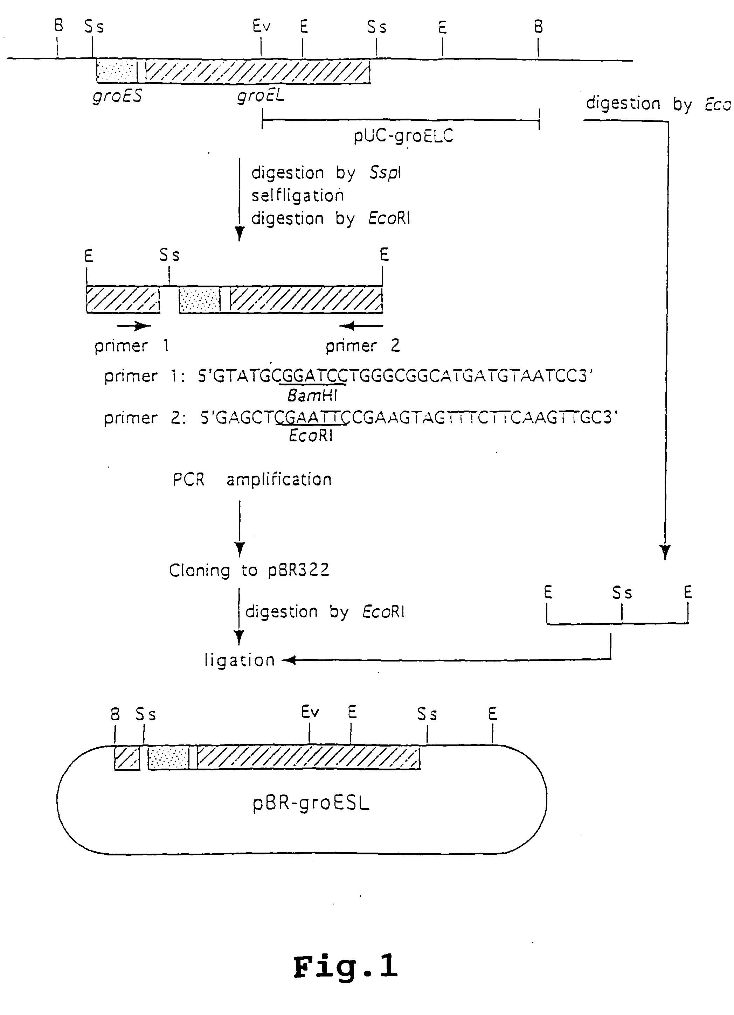

In figure 1, a cloning procedure for the GroESL gene is shown. Chromosomal DNA was isolated by an established method from the starting material, SICI. The chromosomal DNA was digested with SspI and was circularized. The circularized DNA was digested with EcoRI and was subjected to PCR by the use of Primers 1 and 2 having the following sequences: 
1: 5'-GTATGCGGATCCTGGCGGCATGATGTAATCC-3'(SEQ ID No:1)
BamHI
 
2: 5'-GAGCTCGAATTCCGAAGTAGTTTCTTCAAGTTGC-3'(SEQ ID No:2)
EcoRI
 PCR conditions were: 94°C, for 1.5 min; 56°C, for 2.5min.; 72°C, for 3 min.

The DNA amplified by PCR was digested with BamHI and EcoRI, cloned into pBR322, and digested with EcoRI (fragment 1).

On the other hand, pUC-groELC was constructed by digesting chromosomal DNA of SICI with EcoRV1 and BamHI, isolating about a 2.5kb fragment containing a c-terminal region of GroEL, and cloned into pUC19. The pUC-groELC was digested with EcoRI(fragment 2). The EcoRI fragment(fragment 2) was linked to the EcoRI site of the EcoRI digested fragment(fragment 1) above, thereby constructing the plasmid pBR-GroESL having the GroESL gene of SICI.

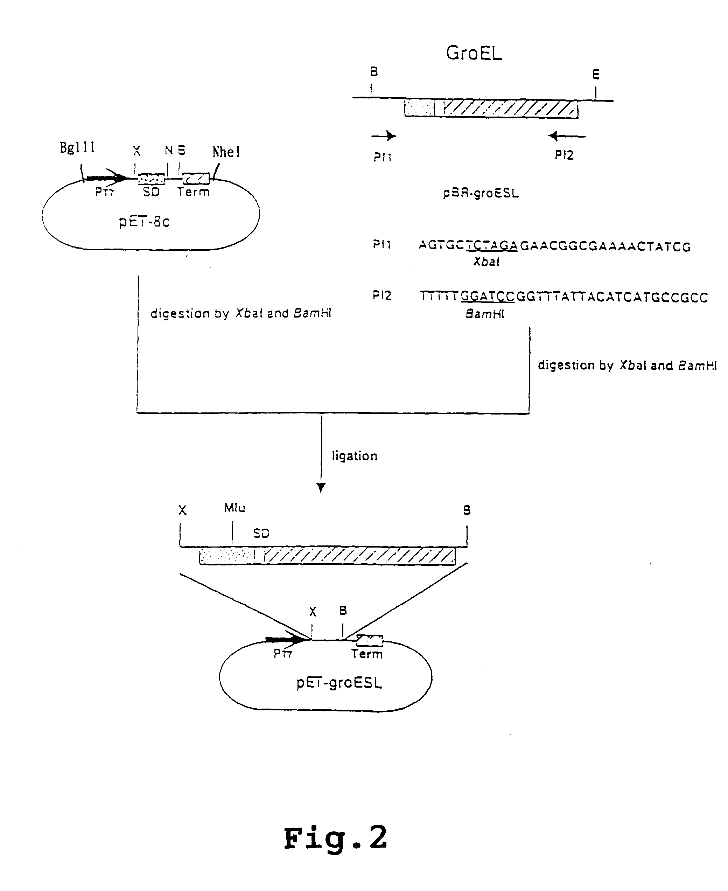

Figure 2 depicts construction of the vector, pET-GroESL. GroESL gene was amplified by PCR using probes P11 and P12 having the following sequences, respectively:

P11:5'-AGTGCTCTAGAGAACGGCGAAAACTATCG-3' (SEQ ID No:3)
XbaI

P12:5'-TTTTTGGATCCGGTTTATTACATCATGCCGCC-3' (SEQ ID No:4)
BamHI

By using the probes above, XbaI site and BamHI sites were introduced into the GroESL gene. The PCR was done under the same condition above. After amplification by PCR, the gene was digested with restriction enzymes XbaI and BamHI, and a XbaI-BamHI fragment containing GroESL gene was recovered.

Plasmid pET-8C which has a T7 promoter and a T7 terminator, was digested with restriction enzymes XbaI and BamHI. To the XbaI-BamHI site, the above XbaI-BamHI fragment containing GroESL gene was ligated, thereby forming the plasmid pET-GroESL.

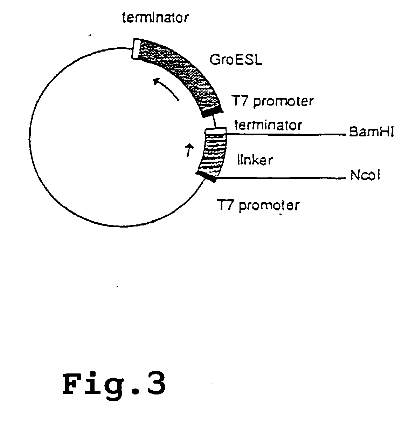

The thus obtained pET-GroESL was digested with BglII, blunt ended, and digested with HindIII. Plasmid pET-8c was digested with a restriction enzyme NheI, blunt ended, and digested with HindIII. These two fragments were ligated and a multi-linker was introduced at the NcoI-BamHI site, thereby forming the expression cassette depicted in Figure 3.

### (Example 2: construction of an expression vector)

This example of an expression vector of the present application which can co-express a molecular chaperon and a single strand peptide Fv (sFv) of anti-gp13O antibody GPX7 (a desired protein).

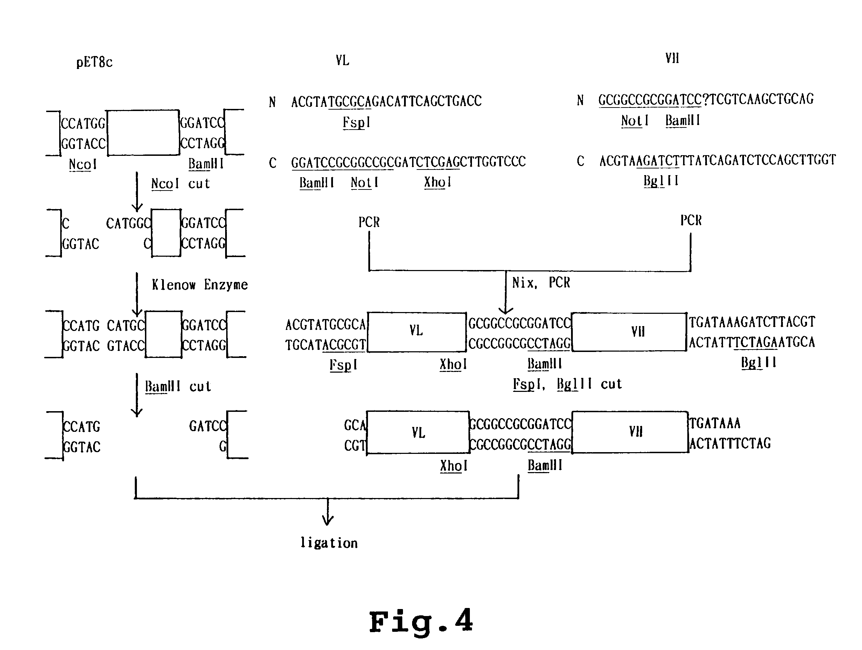

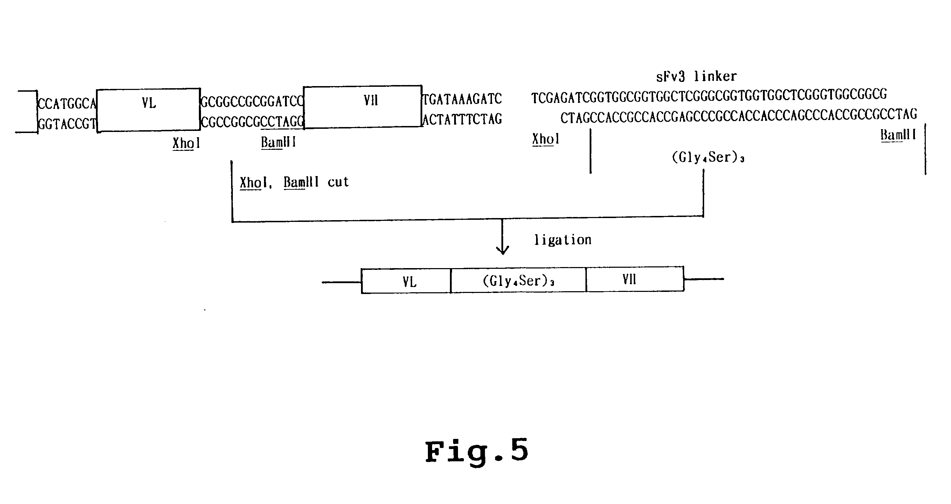

A plasmid pET-sFV having sFv was constructed as depicted in Figures 4 and 5. Plasmid pET-8c was digested with NcoI, blunt ended with a klenow fragment, and digested with BamHI. On the other hand, the VL gene and the VH gene were amplified by PCR using DNA probes as shown in Figure 4, ligated, and digested with the restriction enzymes FspI and BglII. The obtained fragment was ligated to the above BamHI digested plasmid pET-8c. Then, the obtained plasmid was digested with the restriction enzymes XhoI and BamHI, and a sFV3 linker having XhoI and BamHI ends as shown in Figure 5 was ligated to the restriction site, thereby forming the plasmid pET-sFV.

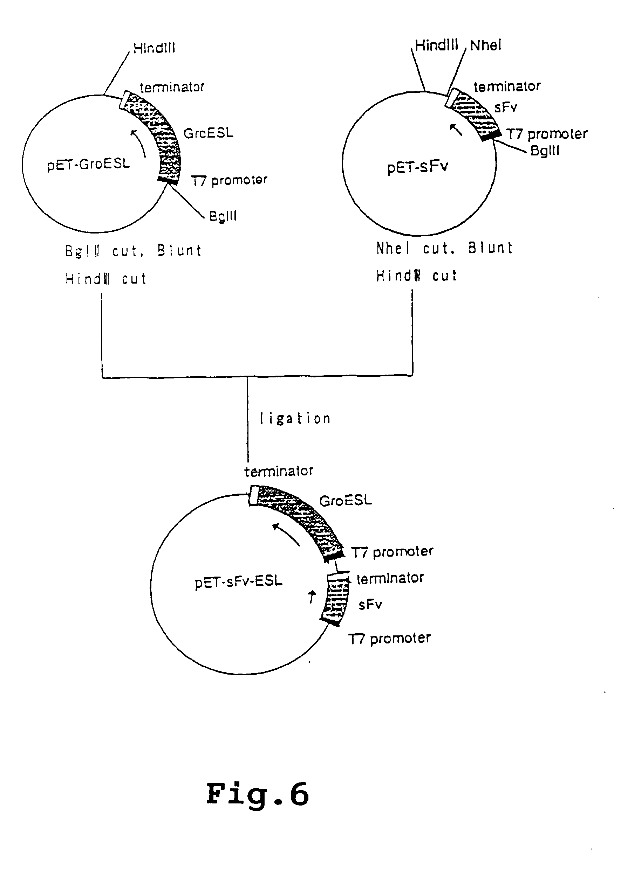

Then, pET-sFV-ESL was constructed by using pET-GroESL and pET-sFV as depicted in Figure 6. Plasmid pET-GroESL constructed in Example 1 was digested with BglII, blunt ended with a klenow fragment, digested with HindIII, and a shorter fragment was recovered. Plasmid pET-sFV was digested with NheI, blunt ended with a klenow fragment, digested with HindIII, and a larger fragment was recovered. These fragments were ligated with T4 DNA ligase to construct a plasmid pEt-sFV-ESL. The pET-sFV-ESL has a T7 promoter which is controlled by a lac operator integrated into the genome of the host cell, and therefore, the expression of the plasmid can be induced by IPTG.

### (Example 3: transformation and expression of sFV)

Escherichia coli (E.coli) BL21(DE3) was inoculated in 40ml LB medium, and cultivated at 37°C for 3 hours. The cells were harvested and treated with 50mM CaCl₂. One micro gram of the pET-sFV-ESL plasmid was added to the cell suspension and the suspension was then treated at 42°C for 2.5 min. The cells were plated on an LB medium containing 50µg/ml ampicillin and cultivated at 37°C for 18 hours, thereby obtaining the derived transformants.

Transformants containing pEt-sFV-ESL plasmid were cultivated in 100ml of NZCYM medium at 37°C. The composition of NZCYM medium is: NZ amine 1%; NaCl 0.5%; yeast extract 0.5%; casamino acid 0.1%; MgSO₄^{·}7H₂O 0.2%; pH 7 with NaOH.

After 2 hours, the transformants was induced by 0.1mM IPTG for 3 hours. After induction, the tranformants were centrifuged, washed with 30mM Tris-NaCl buffer (pH8.0), resuspended in lml of the same buffer, and then lysed by sonication. The lysate was centrifuged to obtain a supernatant fraction (soluble fraction) and precipitate fraction (insoluble fraction).

The insoluble fraction was dissolved with Triton X-100. The dissolved fraction of the precipitate was subjected to SDS-PAGE in order to detect the expressed sFV. As controls, soluble and insoluble fractions from E. coli BL21(DE3) transformed with a plasmid pET-sFV were used. Under microscopic observation, inclusion bodies were not substantially found in transformants having the plasmid pEt-sFV-ESL, however, inclusion bodies were found in transformants having the plasmid pEt-sFV.

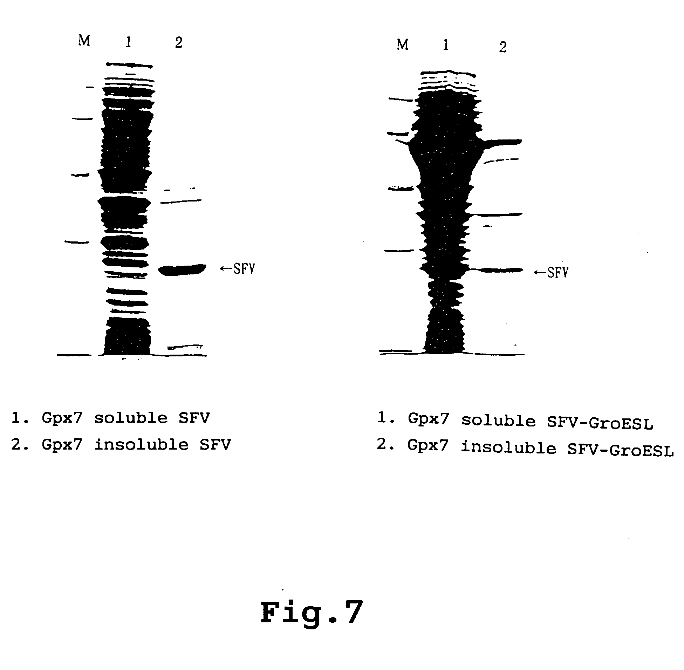

Figure 7 shows a result of an SDS-PAGE of sFV obtained from each transformant. The left column is a control. As clearly shown in the figure, substantially no sFV was found in the soluble fraction but sFV was found in the insoluble fraction. The right column is a case where sFV and molecular chaperon were co-expressed. Almost all sFV was found in the soluble fraction and a small amount of sFV was found in the insoluble fraction.

### (Example 4: cloning of HSP gene from KOD-1)

KOD-1 was cultivated in a 2 litre fermentation jar. KOD-1 was inoculated in 1 litre of 0.5×2216 marine broth medium (2216 marine broth 18.7g/L; PIPES 3.48g/L; CaCl₂. H₂O g/L; 0.4 mL of 0.2% resazurin; 475mL of artificial sea water (NaCl 28.16 g/L; KCl 0.7 g/L; MgCl₂^{·}6H₂O 5.5 g/L; MgSO₄^{·}7H₂O 6.9 g/L) , 500 mL of distilled water, pH7.0) as described in Appl. Environ. Microbiol. 60(12), pp.4559-4566 (1994). The air in the jar was replaced by nitrogen gas and the inner pressure was maintained at 0.1Kg/cm². The culture was grown at 85±1°C for 14 hours, without agitation or bubbling with nitrogen gas. After cultivation, the culture broth (about 1,000ml) was centrifuged at 10,000rpm for 10 min. The cells were harvested.

Chromosomal DNA was extracted according to a well known method and digested with EcoRI and HindIII. Fragments of about 4.5kb were isolated and ligated to pUC18 and transformed to E.coli JM109, thereby preparing the gene library. This library was used to clone a HSP gene of hyperthermophilic archaeon KOD-1. Although a promoter gene of the hyperthermophilic archaeon cannot work well in E.coli, the cloned gene of the Hyperthermophilic archaeon can be expressed since pUC18 has a lac promoter just upstream of the cloning site. Furthermore, since the 16SrRNA binding sequence which is necessary for translating the hyperthermophilic archaeon gene can be functional in E.coli, the cloned hyperthermophilic archaeon gene can be expressed.

Cloning probes were prepared in consideration of the conserved amino acid sequence of the chaperonin genes which are encoded by known HSP genes. The sequences of the probes were: PN: 5'-GGGNGTACCACNATHACNAAYGAYGGNGC-3' (SEQ ID No:5) PC: 5'-GGCATNCCRAARAGGATHGARAAYGC-3'     (SEQ ID No:6) wherein N is one of G, A, T, or C; Y is T or C; H is A, T, or C; and R is G or A.

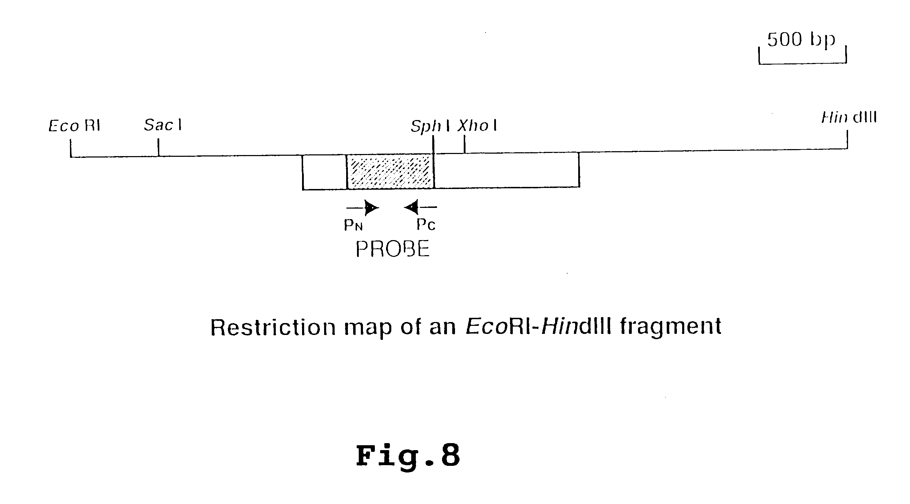

These two probes were used to screen HSP gene of Hyperthermophilic archaeon. Seven positive colonies were selected by colony hybridization from about 1,000 transformants. Southern hybridization was performed as a second screening. Figure 8 shows a restriction enzyme map of the obtained 4.5 kb fragment. The probes were hybridized to a site, which is shown by oblique lines.

PCR was performed using the sequences of ID numbers 5 and 6. DNA sequences were determined by dideoxy chain termination method using a fluorescence labeled primer (AutoRead™, Pharmacia, Upsala, Sweden). The DNA sequence data was analyzed using DNASIS™ (Hitachi Software).

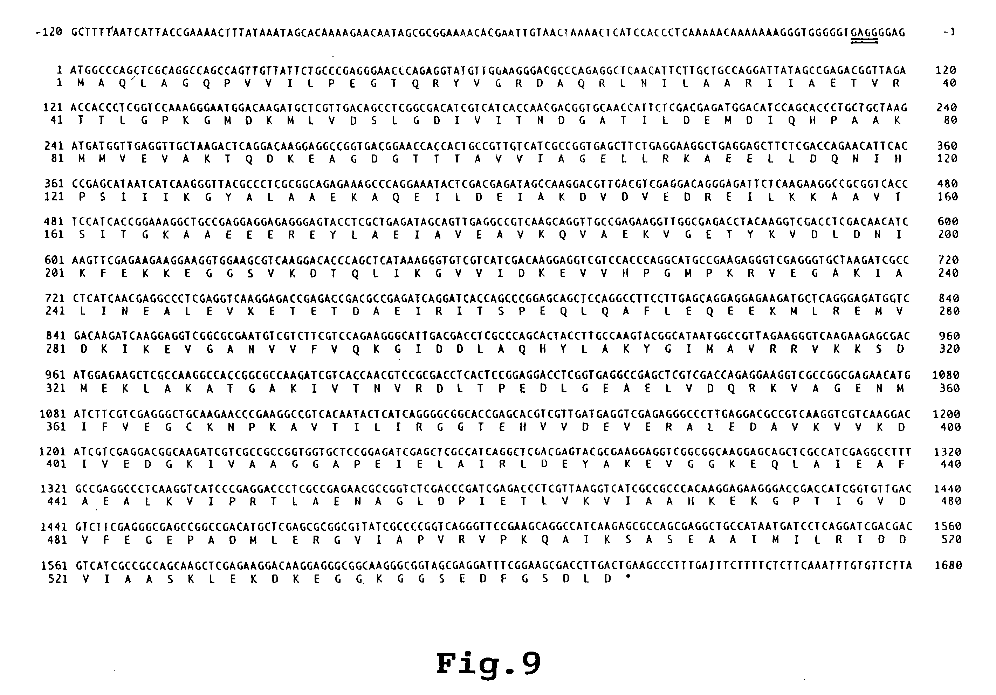

The HSP gene sequence and the deduced amino acid sequence (546 amino acids) of hyperthermophilic archaeon KOD-1 are shown in Figure 9 (SEQ ID No:7). An SD sequence was found upstream of the initiation coden. Figure 1 is a comparison of the homology of the amino acid sequence between HSPs of KOD-1 and other strains.

**Table 1**

| Amino acid sequence comparisons (%) | | | | | | |
|---|---|---|---|---|---|---|
| | TF55 | TCPE mouse | TCPA yeast | TCPA human | *Bs* groEL | *Bs* dnaK |
| *Pk*HSP | 56.3 | 42.8 | 38.4 | 39.4 | 21.1 | 10.0 |
| *Pk*HSP: *Pyrococcus* sp. K0D1 HSP | | | | | | |
| TF55: *Sulfolobus shibatae* thermophile factor 55 | | | | | | |
| TCPE mouse: mouse t-complex protein E unit | | | | | | |
| TCPA yeast: Saccharomyces cerevisiae t-complex protein alpha unit | | | | | | |
| TCPA human: human t-complex protein alpha unit | | | | | | |
| groEL: *Bacillus stearothermophilus* groEL | | | | | | |
| dnAK: *Bacillus stearothermophilus* dnAK | | | | | | |

As shown in Table 1, HSP of KOD-1 has a high amino acid homology of 56.3% with TF55 of Sulfolobus shibatae, and an amino acid homology of 38.4% and 42.8% with the t-complex polypeptide-1 of yeast and mouse, respectively. Only 21.1% homology was found with GroEL of B.stearothermophilus SICI.

### (Example 5: construction of expression cassette of HSP gene of KOD-1)

The primers: 
Pk1: AGGGGCCATGGCCCAGCTCGCAGGCCAGC (SEQ ID NO:8) and
NcoI
 
Pk2: AAAAGGGATCCAAGGTCATCAGTCAAGG (SEQ ID NO:9)
BamHI
 were used as amplification primers for PCR of the HSP gene of KOD-1. The PCR conditions were: 94°C, for 1.5 min; 56°C, for 2.5min.; 72°C, for 3 min.

The obtained gene was digested with NcoI and BamHI and ligated to the NcoI-BamHI site of pET-8C, thereby forming a plasmid pET-KOD Hsp. The plasmid pET-KOD Hsp has a T7 promoter which is controlled by a lac operator integrated into the genome of the host cell; therefore, expression of the plasmid pET-KOD Hsp can be induced by IPTG.

### (Example 6: purification of HSP)

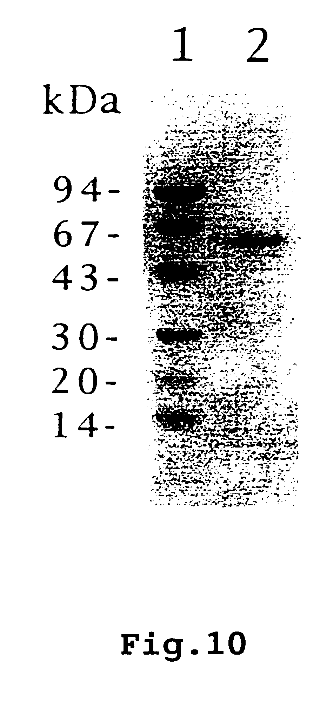

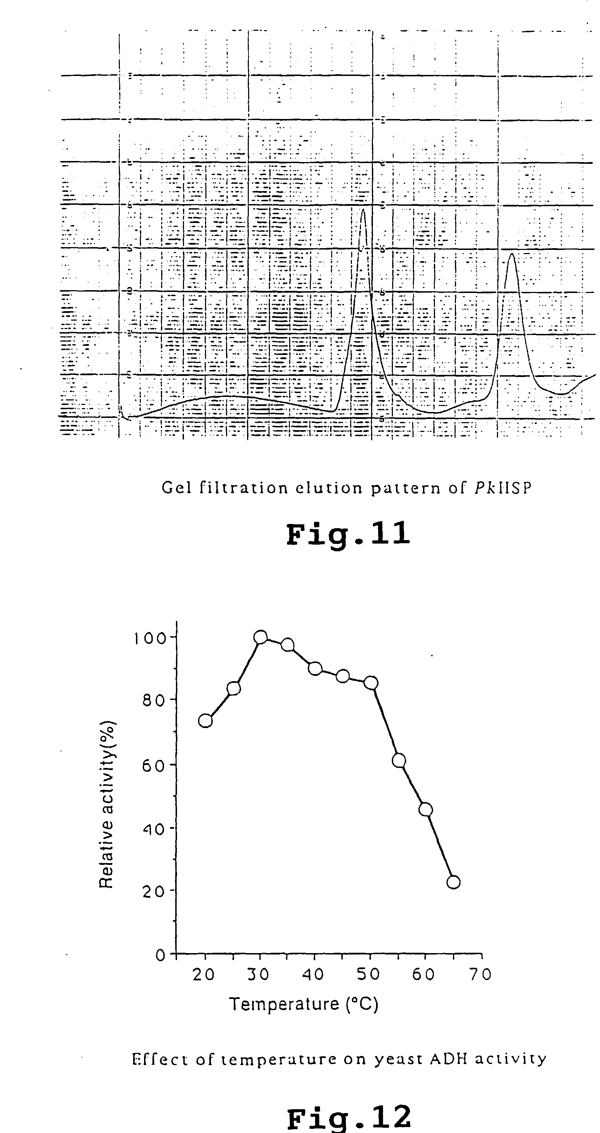

Plasmid pET-KOD Hsp was transformed to E.coli BL21(DE3). Transformants were cultured at 37°C in NZCYM medium. The transformants were induced with 0.1mM IPTG for 3 hours. The cells were then centrifuged, suspended in TE50-1 buffer (50mM Tris HCl, pH8.O, 1mM EDTA), and sonicated at intervals of 30 X 40 seconds and 20 seconds on ice. The treated cells were centrifuged at 4°C at 8,000rpm for 10 minutes and the soluble fraction and insoluble fraction was separated. The soluble fraction was treated at 4°C with 80% ammonium sulfate overnight, and the precipitate was centrifuged at 8,000rpm for 20 min. The precipitate was re-suspended in TE50-1 buffer and dialyzed overnight. The dialyzed fraction was heat treated at 94°C for 20 min. and centrifuged at 12,000rpm for 20 min at 4°C. Proteins were purified by using HiTraP DEAE anion exchange chromatography (FPLC system, Pharmacia, Sweden) with a two-solvent system at a rate of lml/min. Solvent A was 50mM phosphate buffer pH 6.2, and solvent B contained 1.5M NaCl in solvent A. HSP was eluted at 0.5M NaCl and showed a single band of 60KDa in SDS-PAGE (Figure 10). The gel filtration pattern with superdex 200 HR 10/30, FPLC system, Pharmacia) pattern showed a dimer form and (120Kda) and a polymer form (about 950KDa : 16mer)(figure 11).

### (Example 7: Increase of heat stability of alcohol

### dehydrogenase (ADH) by using Heat Shock Protein)

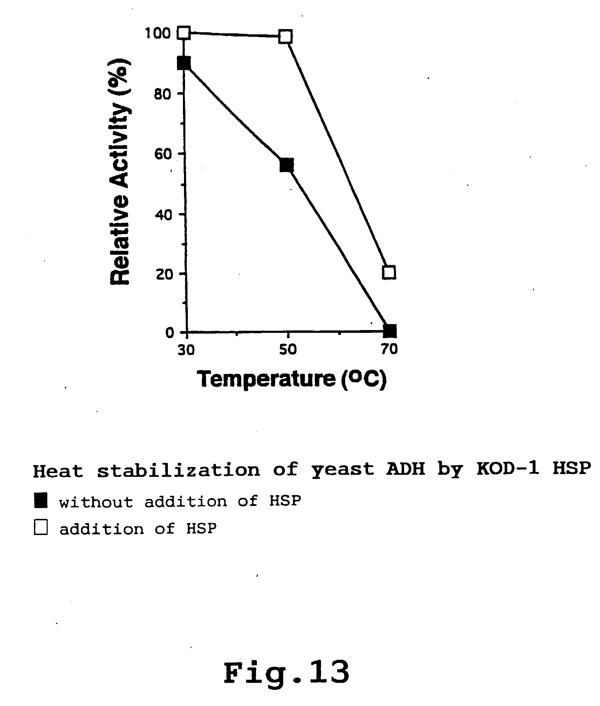

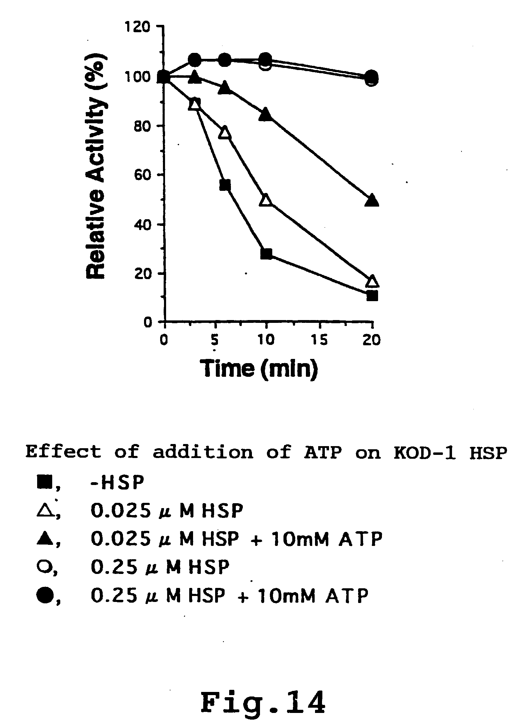

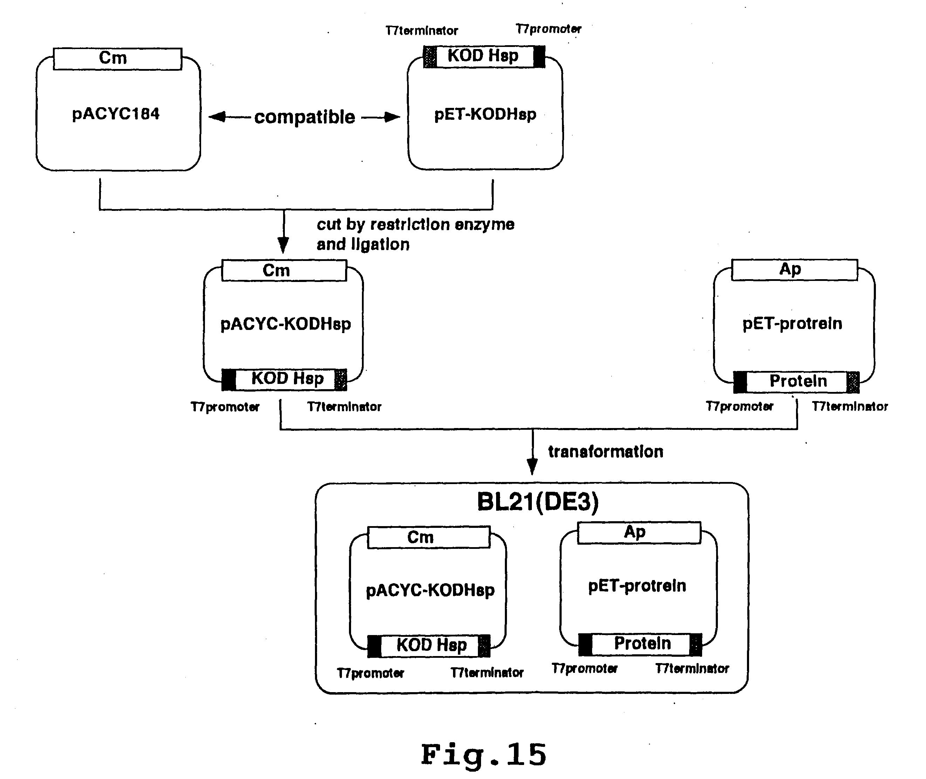

In order to investigate the functions of the molecular chaperon of HSP, the heat stability of ADH was investigated in vitro under heat stress. Figure 12 shows heat stability of ADH in vitro. ADH has a maximum activity at about 30°C, the ADH activity rapidly decreased at about 50°C, and was substantially inactivated at about 70°C. However, in the case where ADH and HSP co-existed, the rate of decrease of ADH activity at high temperature was slowed (Figure 13). This result suggested that HSP binds to thermally unfolded or partially folded ADH. The function of the chaperon, i.e., to maintain the enzymatic active state of ADH in vitro at 50°C, was reproduced. The result is shown in Figure 15. After treatment at 50°C for 20 min., the remaining ADH activity was about 11% without HSP, whereas the remaining ADH activity was about 100% in the presence of HSP (0.25µM). With increase of the HSP concentration, the effect became more remarkable (Figure 14). Further, even with low concentration of HSP, ATP could increase the heat stability of ADH; however, in the presence of a high concentration of HSP, ATP did not affect heat stability. Although both the dimer and polymer form of HSP had chaperon activity (data not shown), the polymer form of HSP could reveal much higher effects than the dimer form.

ADH activity was assayed by monitoring a decrease in absorbance of ethanol dependent NAD at 340nm. ADH activity was expressed as µmoles of NADH produced per minute, calculated with a molar extinction coefficient of 6.22mM cm⁻¹. Standard ADH assay was performed in a mixture at 25°C with 100mM Glycine-KOH buffer(pH 8.8) containing 1mM NAD and 100mM ethanol. A Shimazu UV-visual recording type photometer UV-160 was used to determine the absorbance of 340 nm.

### (Example 8: the construction of plasmids for co-expression and transformation)

Since the effect of HSP on protein stabilization was confirmed in Example 7, the co-expression system (plasmid) was constructed for expressing HSP and the desired protein simultaneously.

Plasmid pET-KOD Hsp as obtained in Example 5 was digested with restriction enzymes BamHI and BglII and a DNA fragment having a T7 promoter-KOD Hsp-T7 terminator was obtained. This fragment was introduced into the BamHI site of pACYC184 which is compatible with a series of pET vectors, thereby constructing a plasmid pACYC-KOD Hsp having a chloramphenicol resistance gene.

The thus obtained plasmid pACYC-KOD Hsp and pET-8C were co-transformed with E.coli BL21(DE3). The transformants were screened for resistance to both ampicillin and chloramphenicol. The HSPs were purified according to the same method in Example 6, showing a single band of MW 60KDa with SDS-PAGE and a polymer form of HSP of about 950KDa was detected. As was confirmed by this result, since co-transformation of plasmid pACYC-KOD Hsp and series of pET vectors are possible, it is possible to co-express the HSP and the desired protein by incorporating the gene of the desired protein into a cloning site of pET-8c, as shown figure 15.

### (Example 9: co-expression of HSP and neutral amylase of KOD-1)

In the co-expression system obtained in Example 8, HSP and neutrals amylase of KOD-1 were co-expressed. The neutral amylase of KOD-1 was screened as follows:

Chromosomal DNA of KOD-1 as obtained in Example 4 was digested with EcoRI. Fragments of about 7.5kb were isolated and inserted into the EcoRI site of pUC18, transformed to E.coli JM109, and the gene library was prepared. The transformants were grown on a starch azure agar (L-agar containing a final concentration of starch azure 0.25% : amylase activity indicating medium) containing ampicillin, heat treated at 60°C overnight, and a characteristic halo-forming colony was selected. The amylase obtained from this colony was confirmed to be neutral amylase by its optimum pH of 5.0 to 7.0.

The cloned DNA fragment was isolated from the transformant and its DNA sequence was determined. The DNA was amplified by PCR using the following primers:

SD2: 5'-TGGTACCATGGCAAAGTATTCCGAACTCGA-3' (SEQ ID No:10)
NcoI

E5: 5'-CGGATCCGATATCAGCTATGACCTTTA-3'    (SEQ ID No:11)
BamHI

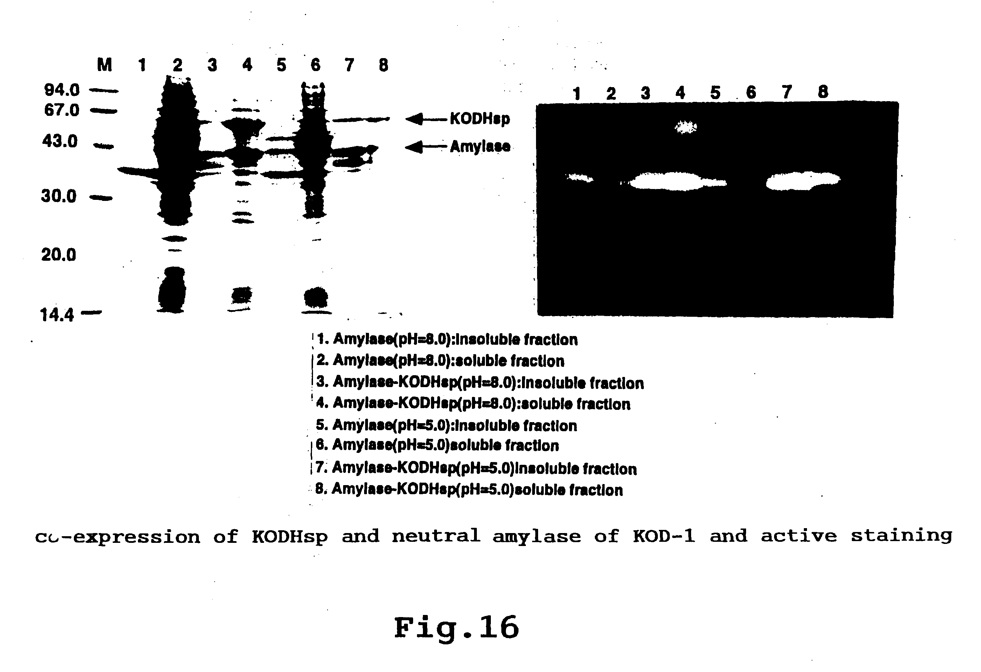

The neutral amylase gene obtained was digested with NcoI and BamHI, incorporated into the NcoI and BamHI site of pET-8c, thereby constructing a plasmid pET-NAmy. Plasmids pACYC-KOD Hsp and pET-NAmy are co-transformed with E.coli BL21(DE3), and a strain resistant to both ampicillin and chloramphenicol were selected. The transformants were cultured in NZCYM medium in the same manner as in Example 6 and induced by IPTG for 3 hours. As a control, E.coli BL21(DE3) transformed with pET-NAmy alone was used. The results are shown in Figure 16. The neutral amylase aggregates at pH 5.0 but is soluble at pH 8.0. The transformants were sonicated at pH 5.0 and pH 8.0, and the cells were fractionated. SDS-PAGE and active staining showed an increase of amylase production per cell in the co-transformed cells at pH 5.0. Further, an increase of amylase expression in the soluble fraction was recognized at pH 8.0.

### (Example 10: co-expression of cobyric acid synthetase (CobQ) and HSP)

When CobQ of KOD-1 is expressed in E.coli, soluble CobQ and insoluble inclusion body of CobQ are equally expressed.

When analyzing the genome of KOD-1, a comparison with sequences of Salmonella and Pseudomonas revealed that the CobQ gene was included in 4.5Kb HindIII fragment. The CobQ gene was amplified by PCR using the following two primers:

COB-1: 5'-GTGACCATGGGAAAGGCGCTGATGGTTCA (SEQ ID No:12)
NcoI

COB-2:5'-CTAGGATCCAAGTCTCTGGATTATGTACTGGA (SEQ ID No:13)
BamHI

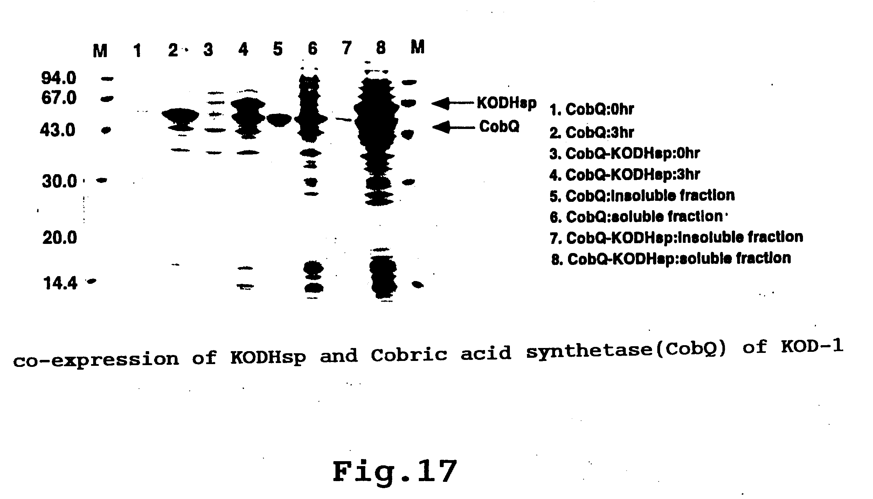

The obtained CobQ gene was digested with NcoI and BamHI, cloned into NcoI-BamHI site of pET-8c, thereby constructing a plasmid pET-CobQ. Plasmid pACYC-KOD Hsp and pET-CobQ were co-transformed to E.coli BL21(DE3), and ampicillin and chloramphenicol resistant transformants were selected. The transformants were cultivated in NZCYM medium as described in Example 6 and induced by IPTG for 3 hours. As a control, E.coli BL21(DE3) transformed with pET-CobQ alone was used. The results are shown in Figure 17. As can be seen in the figure, the number of insoluble inclusion bodies of CobQ decreased when co-expressed with HSP and the expression of soluble CobQ was increased.

### (Example 11: co-expression of sFV and HSP)

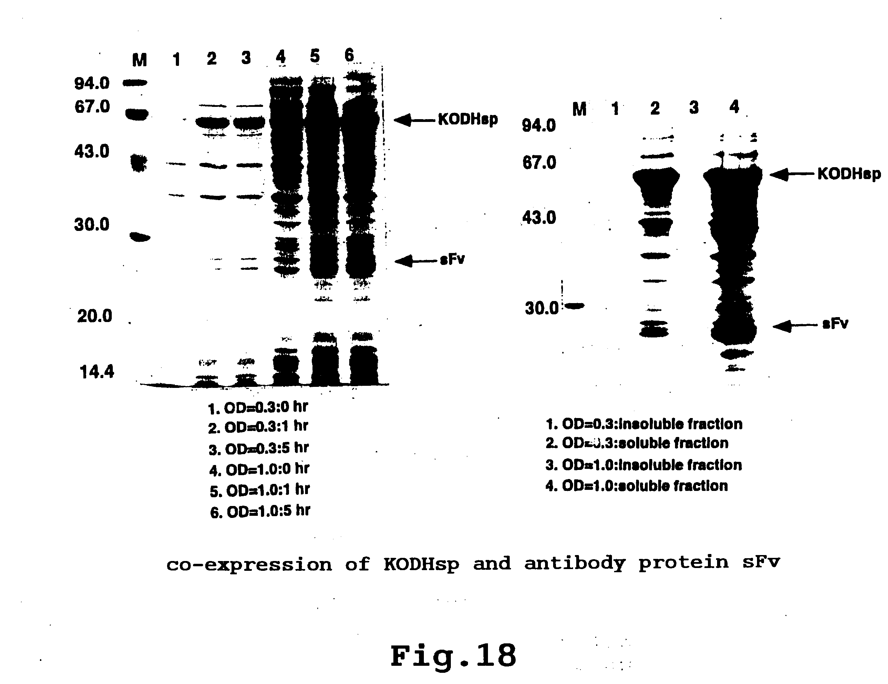

Plasmid pACYC-KOD Hsp and plasmid pET-sFV prepared in Example 2 were co-transformed to E.coli BL21(DE3). The transformant was cultured in NZCYM medium. IPTG was added when O.D.660nm of the culture medium reached 0.3 or 1.0, and the culture was induced for 1 or 5 hours. By co-expression with HSP, sFV was produced as a soluble fraction (Figure 18).

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

Sequence Listing
SEQ ID NO:1
LENGTH:32
SEQUENCE TYPE: Nucleic acid
STRANDNESS: Single
TOPOLOGY: Linear
MOLECULAR TYPE: Other nucleic acid, synthetic DNA
SEQUENCE:
GTATG CGGAT CCTGG GCGGC ATGAT GTAAT CC 32

SEQ ID NO:2
LENGTH:34
SEQUENCE TYPE: Nucleic acid
STRANDNESS: Single
TOPOLOGY: Linear
MOLECULAR TYPE: Other nucleic acid, synthetic DNA
SEQUENCE:
GAGCT CGAAT TCCGA AGTAG TTTCT TCAAG TTGC 34

SEQ ID NO:3

LENGTH:29
SEQUENCE TYPE: Nucleic acid
STRANDNESS: Single
TOPOLOGY: Linear
MOLECULAR TYPE: Other nucleic acid, synthetic DNA
SEQUENCE:
AGTGC TCTAG AGAAC GGCGA AAACT ATCG 29

SEQ ID NO:4
LENGTH:32
SEQUENCE TYPE: Nucleic acid
STRANDNESS: Single
TOPOLOGY: Linear
MOLECULAR TYPE: Other nucleic acid, synthetic DNA
SEQUENCE:
TTTTT GGATC CGGTT TATTA CATCA TGCCG CC 32

SEQ ID NO:5
LENGTH:29
SEQUENCE TYPE: Nucleic acid
STRANDNESS: Single
TOPOLOGY: Linear
MOLECULAR TYPE: Other nucleic acid, synthetic DNA
SEQUENCE:
GGGNG TACCA CNATH ACNAA YGAYG GNGC 29

SEQ ID NO:6
LENGTH:26
SEQUENCE TYPE: Nucleic acid
STRANDNESS: Single
TOPOLOGY: Linear
MOLECULAR TYPE: Other nucleic acid, synthetic DNA
SEQUENCE:

GGCAT NCCRA ARAGG ATHGA RAAYG C 26

SEQ ID NO:7
LENGTH:1800
SEQUENCE TYPE: Nucleic acid
STRANDNESS: Both
TOPOLOGY: Unknown
MOLECULAR TYPE: Genomic DNA
SEQUENCE:
GCTTTTAATC ATTACCGAAA ACTTTATAAA TAGCACAAAA -81
GAACAATAGC GCGGAAAACA CGAATTGTAA CTAAAACTCA -41
TCCACCCTCA AAAACAAAAA AAGGGTGGGG GTGAGGGGAG ATG GCC 6
Met Ala
1
CAG CTC GCA GGC CAG CCA GTT GTT ATT CTG CCC GAG GGA 45
Gln Leu Ala Gly Gln Pro Val Val Ile Leu Pro Glu Gly
        5                   10                  15
ACC CAG AGG TAT GTT GGA AGG GAC GCC CAG AGG CTC AAC 84
Thr Gln Arg Tyr Val Gly Arg Asp Ala Gln Arg Leu Asn
                20                  25
ATT CTT GCT GCC AGG ATT ATA GCC GAG ACG GTT AGA ACC 123
Ile Leu Ala Ala Arg Ile Ile Ala Glu Thr Val Arg Thr
    30                  35                  40
ACC CTC GGT CCA AAG GGA ATG GAC AAG ATG CTC GTT GAC 162
Thr Leu Gly Pro Lys Gly Met Asp Lys Met Leu Val Asp
            45                  50
AGC CTC GGC GAC ATC GTC ATC ACC AAC GAC GGT GCA ACC 201
Ser Leu Gly Asp Ile Val Ile Thr Asn Asp Gly Ala Thr
55                  60                  65
ATT CTC GAC GAG ATG GAC ATC CAG CAC CCT GCT GCT AAG 240
Ile Leu Asp Glu Met Asp Ile Gln His Pro Ala Ala Lys
        70                  75                  80
ATG ATG GTT GAG GTT GCT AAG ACT CAG GAC AAG GAG GCC 279

Met Met Val Glu Val Ala Lys Thr Gln Asp Lys Glu Ala
                85                  90
GGT GAC GGA ACC ACC ACT GCC GTT GTC ATC GCC GGT GAG   318
Gly Asp Gly Thr Thr Thr Ala Val Val Ile Ala Gly Glu
    95                  100                 105
CTT CTG AGG AAG GCT GAG GAG CTT CTC GAC CAG AAC ATT   357
Leu Leu Arg Lys Ala Glu Glu Leu Leu Asp Gln Asn Ile
            110                 115
CAC CCG AGC ATA ATC ATC AAG GGT TAC GCC CTC GCG GCA   396
His Pro Ser Ile Ile Ile Lys Gly Tyr Ala Leu Ala Ala
120                 125                 130
GAG AAA GCC CAG GAA ATA CTC GAC GAG ATA GCC AAG GAC   435
Glu Lys Ala Gln Glu Ile Leu Asp Glu Ile Ala Lys Asp
        135                 140                 145
GTT GAC GTC GAG GAC AGG GAG ATT CTC AAG AAG GCC GCG   474
Val Asp Val Glu Asp Arg Glu Ile Leu Lys Lys Ala Ala
                150                 155
GTC ACC TCC ATC ACC GGA AAG GCT GCC GAG GAG GAG AGG   513
Val Thr Ser Ile Thr Gly Lys Ala Ala Glu Glu Glu Arg
    160                 165                 170
GAG TAC CTC GCT GAG ATA GCA GTT GAG GCC GTC AAG CAG   552
Glu Tyr Leu Ala Glu Ile Ala Val Glu Ala Val Lys Gln
            175                 180
GTT GCC GAG AAG GTT GGC GAG ACC TAC AAG GTC GAC CTC   591
Val Ala Glu Lys Val Gly Glu Thr Tyr Lys Val Asp Leu
185                 190                 195
GAC AAC ATC AAG TTC GAG AAG AAG GAA GGT GGA AGC GTC   630
Asp Asn Ile Lys Phe Glu Lys Lys Glu Gly Gly Ser Val
        200                 205                 210
AAG GAC ACC CAG CTC ATA AAG GGT GTC GTC ATC GAC AAG   669
Lys Asp Thr Gln Leu Ile Lys Gly Val Val Ile Asp Lys
                215                 220
GAG GTC GTC CAC CCA GGC ATG CCG AAG AGG GTC GAG GGT   708

Glu Val Val His Pro Gly Met Pro Lys Arg Val Glu Gly
225                 230                 235
GCT AAG ATC GCC CTC ATC AAC GAG GCC CTC GAG GTC AAG   747
Ala Lys Ile Ala Leu Ile Asn Glu Ala Leu Glu Val Lys
        240                 245
GAG ACC GAG ACC GAC GCC GAG ATC AGG ATC ACC AGC CCG   786
Glu Thr Glu Thr Asp Ala Glu Ile Arg Ile Thr Ser Pro
250                 255                 260
GAG CAG CTC CAG GCC TTC CTT GAG CAG GAG GAG AAG ATG   825
Glu Gln Leu Gln Ala Phe Leu Glu Gln Glu Glu Lys Met
        265                 270                 275
CTC AGG GAG ATG GTC GAC AAG ATC AAG GAG GTC GGC GCG   864
Leu Arg Glu Met Val Asp Lys Ile Lys Glu Val Gly Ala
                280                 285
AAT GTC GTC TTC GTC CAG AAG GGC ATT GAC GAC CTC GCC   903
Asn Val Val Phe Val Gln Lys Gly Ile Asp Asp Leu Ala
    290                 295                 300
CAG CAC TAC CTT GCC AAG TAC GGC ATA ATG GCC GTT AGA   942
Gln His Tyr Leu Ala Lys Tyr Gly Ile Met Ala Val Arg
            305                 310
AGG GTC AAG AAG AGC GAC ATG GAG AAG CTC GCC AAG GCC   981
Arg Val Lys Lys Ser Asp Met Glu Lys Leu Ala Lys Ala
315                 320                 325
ACC GGC GCC AAG ATC GTC ACC AAC GTC CGC GAC CTC ACT  1020
Thr Gly Ala Lys Ile Val Thr Asn Val Arg Asp Leu Thr
        330                 335                 340
CCG GAG GAC CTC GGT GAG GCC GAG CTC GTC GAC CAG AGG  1059
Pro Glu Asp Leu Gly Glu Ala Glu Leu Val Asp Gln Arg
                345                 350
AAG GTC GCC GGC GAG AAC ATG ATC TTC GTC GAG GGC TGC  1098
Lys Val Ala Gly Glu Asn Met Ile Phe Val Glu Gly Cys
    355                 360                 365
AAG AAC CCG AAG GCC GTC ACA ATA CTC ATC AGG GGC GGC  1137

Lys Asn Pro Lys Ala Val Thr Ile Leu Ile Arg Gly Gly
            370                 375
ACC GAG CAC GTC GTT GAT GAG GTC GAG AGG GCC CTT GAG 1176
Thr Glu His Val Val Asp Glu Val Glu Arg Ala Leu Glu
380                 385                 390
GAC GCC GTC AAG GTC GTC AAG GAC ATC GTC GAG GAC GGC 1215
Asp Ala Val Lys Val Val Lys Asp Ile Val Glu Asp Gly
        395                 400                 405
AAG ATC GTC GCC GCC GGT GGT GCT CCG GAG ATC GAG CTC 1254
Lys Ile Val Ala Ala Gly Gly Ala Pro Glu Ile Glu Leu
                410                 415
GCC ATC AGG CTC GAC GAG TAC GCG AAG GAG GTC GGC GGC 1293
Ala Ile Arg Leu Asp Glu Tyr Ala Lys Glu Val Gly Gly
    420                 425                 430
AAG GAG CAG CTC GCC ATC GAG GCC TTT GCC GAG GCC CTC 1332
Lys Glu Gln Leu Ala Ile Glu Ala Phe Ala Glu Ala Leu
            435                 440
AAG GTC ATC CCG AGG ACC CTC GCC GAG AAC GCC GGT CTC 1371
Lys Val Ile Pro Arg Thr Leu Ala Glu Asn Ala Gly Leu
445                 450                 455
GAC CCG ATC GAG ACC CTC GTT AAG GTC ATC GCC GCC CAC 1410
Asp Pro Ile Glu Thr Leu Val Lys Val Ile Ala Ala His
        460                 465                 470
AAG GAG AAG GGA CCG ACC ATC GGT GTT GAC GTC TTC GAG 1449
Lys Glu Lys Gly Pro Thr Ile Gly Val Asp Val Phe Glu
                475                 480
GGC GAG CCG GCC GAC ATG CTC GAG CGC GGC GTT ATC GCC 1488
Gly Glu Pro Ala Asp Met Leu Glu Arg Gly Val Ile Ala
    485                 490                 495
CCG GTC AGG GTT CCG AAG CAG GCC ATC AAG AGC GCC AGC 1527
Pro Val Arg Val Pro Lys Gln Ala Ile Lys Ser Ala Ser
            500                 505
GAG GCT GCC ATA ATG ATC CTC AGG ATC GAC GAC GTC ATC 1566

Glu Ala Ala Ile Met Ile Leu Arg Ile Asp Asp Val Ile
510                    515                    520
GCC GCC AGC AAG CTC GAG AAG GAC AAG GAG GGC GGC AAG  1605
Ala Ala Ser Lys Leu Glu Lys Asp Lys Glu Gly Gly Lys
        525                    530                    535
GGC GGT AGC GAG GAT TTC GGA AGC GAC CTT GAC          1638
Gly Gly Ser Glu Asp Phe Gly Ser Asp Leu Asp
                540                    545 546
TGAAGCCCTT TGATTTCTTT TCTCTTCAAA TTTGTGTTCT TA       1680

SEQ ID NO:8
LENGTH:29
SEQUENCE TYPE: Nucleic acid
STRANDNESS: Single
TOPOLOGY: Linear
MOLECULAR TYPE: Other nucleic acid, synthetic DNA
SEQUENCE:
AGGGG CCATG GCCCA GCTCG CAGGC CAGC                    29

SEQ ID NO:9
LENGTH:28
SEQUENCE TYPE: Nucleic acid
STRANDNESS: Single
TOPOLOGY: Linear
MOLECULAR TYPE: Other nucleic acid, synthetic DNA
SEQUENCE:
AAAAG GGATC CAAGG TCATC AGTCA AGG                     28

SEQ ID NO:10
LENGTH:30
SEQUENCE TYPE: Nucleic acid
STRANDNESS: Single

TOPOLOGY: Linear
MOLECULAR TYPE: Other nucleic acid, synthetic DNA
SEQUENCE:
TGGTA CCATG GCAAA GTATT CCGAA CTCGA 30

SEQ ID NO:11
LENGTH:27
SEQUENCE TYPE: Nucleic acid
STRANDNESS: Single
TOPOLOGY: Linear
MOLECULAR TYPE: Other nucleic acid, synthetic DNA
SEQUENCE:
CGGAT CCGAT ATCAG CTATG ACCTT TA 27

SEQ ID NO:12
LENGTH:29
SEQUENCE TYPE: Nucleic acid
STRANDNESS: Single
TOPOLOGY: Linear
MOLECULAR TYPE: Other nucleic acid, synthetic DNA
SEQUENCE:
GTGAC CATGG GAAAG GCGCT GATGG TTCA 29

SEQ ID NO:13
LENGTH:32
SEQUENCE TYPE: Nucleic acid
STRANDNESS: Single
TOPOLOGY: Linear
MOLECULAR TYPE: Other nucleic acid, synthetic DNA
SEQUENCE:
CTAGG ATCCA AGTCT CTGGA TTATG TACTG GA 32

## Claims

1. An expression cassette which can express a desired protein in a cell, wherein the cassette comprises a sequence in which a gene encoding a molecular chaperon is operably linked to a first promoter and an insertion site into which a gene encoding the desired protein can be inserted.

2. An expression cassette as claimed in claim 1, wherein the cell is a bacterial cell and the desired protein is expressed in a soluble form when, in the absence of the molecular chaperon the desired protein would be expressed as an insoluble protein.

3. An expression cassette as claimed in any of the preceding claims, wherein the cassette has a second promoter, which is upstream of the insertion site.

4. An expression cassette as claimed in any of the preceding claims wherein the cassette has a terminator sequence downstream of the gene encoding the molecular chaperon and downstream of the insertion site.

5. An expression cassette as claimed in any of the preceding claims wherein the gene encoding the molecular chaperon is a heat shock protein gene of a hyperthermophilic archaeon KOD-1.

6. An expression cassette as claimed in any of claims 1 to 4, wherein the gene encoding the molecular chaperon is a GroESL gene of Bacillus stearothermophilus SICI.

7. An expression cassette as claimed in any of the preceding claims wherein the first and/or the second promoter is a T7 promoter.

8. An expression vector comprising an expression cassette as claimed in any of claims 1 to 7, in which a gene encoding the desired protein is operably incorporated into the insertion site.

9. A cell which can express a desired protein, wherein the cell is transformed with an expression cassette as claimed in any of claims 1 to 7 or an expression vector comprising the expression cassette as claimed in claim 8.

10. A cell which can express a desired protein, wherein the host cell is co-transformed with a vector which can express a gene encoding a molecular chaperon and a vector which can express a sequence encoding the desired protein.

11. The cell of claim 10, wherein the cell is a bacterial cell and the gene encoding the molecular chaperon is a heat shock protein gene of a hyperthermophilic archaeon KOD-1.

12. The cell of claim 10, wherein the cell is a bacterial cell and the gene encoding the molecular chaperon is a GroESL gene of Bacillus stearothermophilus SICI.

13. A method of expressing a desired protein, the method comprising a step of culturing a cell which can co-express a gene encoding a molecular chaperon and a gene encoding the desired protein.

14. A method as claimed in claim 13, wherein the cell is transformed with a vector having a gene encoding a molecular chaperon which is operably linked to a first promoter and a gene encoding the desired protein which is operably linked to a second promoter.

15. The method as claimed in claim 13, wherein the cell is co-transformed with a vector which can express a gene encoding a molecular chaperon and a vector which can express a sequence encoding the desired protein.

16. A method as claimed in claim 13, wherein the cell is a bacterial cell and the desired protein is expressed in a soluble form when, in the absence of the molecular chaperon the desired protein would be expressed as an insoluble protein.

17. A method as claimed in claim 16, wherein the gene encoding the molecular chaperon is a heat shock protein gene of a hyperthermophilic archaeon KOD-1.

18. A method as claimed in claim 16, wherein the gene encoding the molecular chaperon is a GroESL gene of Bacillus stearothermophilus SICI.

19. A method of expressing a desired protein comprising:
culturing a cell comprising an expression cassette as claimed in any of claims 1 to 7 or an expression vector as claimed in claim 8; co-expressing the molecular chaperon and the desired protein;
heating the cell culture or a fraction containing the desired protein;
separating an insoluble fraction; and
recovering the desired protein.

20. A method as claimed in claim 19, wherein the cell is transformed with a vector in which the gene encoding the molecular chaperon is operably linked to the first promoter and the gene encoding the desired protein is operably linked to the second promoter.

21. A method of expressing a desired protein comprising:
culturing a cell which has been co-transformed with a vector which can express a gene encoding a molecular chaperon and a vector which can express a sequence encoding a desired protein, co-expressing the molecular chaperon and the desired protein; heating the cell culture or a fraction containing the desired protein; separating an insoluble fraction and recovering the desired protein.

22. A method of changing a heat labile protein to a heat stable protein comprising mixing the heat labile protein with a heat stable molecular chaperon.

23. A method of purifying a heat labile protein comprising:
mixing the heat labile protein with a heat stable molecular chaperon; and
heating the mixture.

24. A KOD-1 heat shock protein comprising an amino acid sequence of SEQ ID NO: 7.

25. A gene encoding a KOD-1 heat shock protein comprising an amino acid sequence of SEQ ID NO: 7.


SEQUENCE LISTING

(1) GENERAL INFORMATION:

(i) APPLICANT:
(A) NAME: IMANAKA, TADAYUKI
(B) STREET: 2-28-11 FUJISHIRO-DAI
(C) CITY: OSAKA
(E) COUNTRY: JAPAN
(F) POSTAL CODE (ZIP): .

(ii) TITLE OF INVENTION: A METHOD FOR PRODUCTION OF PROTEIN USING
MOLECULAR CHAPERON.

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96306713.7
(vi) PRIOR APPLICATION DATA:
(A) APPLICATION NUMBER: JP 7-237176
(B) FILING DATE: 14-SEP-1995

(vi) PRIOR APPLICATION DATA:
(A) APPLICATION NUMBER: JP 8-228252
(B) FILING DATE: 29-AUG-1996

(2) INFORMATION FOR SEQ ID NO: 1:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 1:

GTATGCGGAT CCTGGGCGGC ATGATGTAAT CC 32

(2) INFORMATION FOR SEQ ID NO: 2:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 2:

GAGCTCGAAT TCCGAAGTAG TTTCTTCAAG TTGC
34

(2) INFORMATION FOR SEQ ID NO: 3:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 3:

AGTGCTCTAG AGAACGGCGA AAACTATCG 29

(2) INFORMATION FOR SEQ ID NO: 4:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 4:

TTTTTGGATC CGGTTTATTA CATCATGCCG CC 32

(2) INFORMATION FOR SEQ ID NO: 5:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 5:

GGGNGTACCA CNATHACNAA YGAYGGNGC 29

(2) INFORMATION FOR SEQ ID NO: 6:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 6:

GGCATNCCRA ARAGGATHGA RAAYGC 26

(2) INFORMATION FOR SEQ ID NO: 7:

(i) SEQUENCE CHARACTERISTICS:
(A) LENGTH: 1800 base pairs
(B) TYPE: nucleic acid
(C) STRANDEDNESS: both
(D) TOPOLOGY: unknown

(ii) MOLECULE TYPE: DNA (genomic)

(ix) FEATURE:
(A) NAME/KEY: CDS
(B) LOCATION:121..1761

(xi) SEQUENCE DESCRIPTION: SEQ ID NO: 7:

GCTTTTAATC ATTACCGAAA ACTTTATAAA TAGCACAAAA
GAACAATAGC GCGGAAAACA 60

CGAATTGTAA CTAAAACTCA TCCACCCTCA AAAACAAAAA
AAGGGTGGGG GTGAGGGGAG 120

ATG GCC CAG CTC GCA GGC CAG CCA GTT GTT ATT CTG CCC GAG
GGA ACC 168
Met Ala Gln Leu Ala Gly Gln Pro Val Val Ile Leu Pro Glu Gly Thr
1               5                   10                  15

CAG AGG TAT GTT GGA AGG GAC GCC CAG AGG CTC AAC ATT CTT
GCT GCC 216
Gln Arg Tyr Val Gly Arg Asp Ala Gln Arg Leu Asn Ile Leu Ala Ala
            20                  25                  30

AGG ATT ATA GCC GAG ACG GTT AGA ACC ACC CTC GGT CCA AAG
GGA ATG 264

Arg Ile Ile Ala Glu Thr Val Arg Thr Thr Leu Gly Pro Lys Gly Met
        35                  40                  45

GAC AAG ATG CTC GTT GAC AGC CTC GGC GAC ATC GTC ATC ACC
AAC GAC 312
Asp Lys Met Leu Val Asp Ser Leu Gly Asp Ile Val Ile Thr Asn Asp
    50                  55                  60

GGT GCA ACC ATT CTC GAC GAG ATG GAC ATC CAG CAC CCT GCT
GCT AAG 360
Gly Ala Thr Ile Leu Asp Glu Met Asp Ile Gln His Pro Ala Ala Lys
65                  70                  75                  80

ATG ATG GTT GAG GTT GCT AAG ACT CAG GAC AAG GAG GCC GGT
GAC GGA 408
Met Met Val Glu Val Ala Lys Thr Gln Asp Lys Glu Ala Gly Asp Gly
                85                  90                  95

ACC ACC ACT GCC GTT GCC ATC GCC GGT GAG CTT CTG AGG AAG
GCT GAG 456
Thr Thr Thr Ala Val Ala Ile Ala Gly Glu Leu Leu Arg Lys Ala Glu
            100                 105                 110

GAG CTT CTC GAC CAG AAC ATT CAC CCG AGC ATA ATC ATC AAG
GGT TAC 504
Glu Leu Leu Asp Gln Asn Ile His Pro Ser Ile Ile Ile Lys Gly Tyr
        115                 120                 125

GCC CTC GCG GCA GAG AAA GCC CAG GAA ATA CTC GAC GAG ATA
GCC AAG 552
Ala Leu Ala Ala Glu Lys Ala Gln Glu Ile Leu Asp Glu Ile Ala Lys
    130                 135                 140

GAC GTT GAC GTC GAG GAC AGG GAG ATT CTC AAG AAG GCC GCG
GTC ACC 600
Asp Val Asp Val Glu Asp Arg Glu Ile Leu Lys Lys Ala Ala Val Thr
145                 150                 155                 160

TCC ATC ACC GGA AAG GCT GCC GAG GAG GAG AGG GAG TAC CTC
GCT GAG 648
Ser Ile Thr Gly Lys Ala Ala Glu Glu Glu Arg Glu Tyr Leu Ala Glu
                165                 170                 175

ATA GCA GTT GAG GCC GTC AAG CAG GTT GCC GAG AAG GTT GGC
GAG ACC      696
Ile Ala Val Glu Ala Val Lys Gln Val Ala Glu Lys Val Gly Glu Thr
        180                185                190

TAC AAG GTC GAC CTC GAC AAC ATC AAG TTC GAG AAG AAG GAA
GGT GGA      744
Tyr Lys Val Asp Leu Asp Asn Ile Lys Phe Glu Lys Lys Glu Gly Gly
      195                200                205

AGC GTC AAG GAC ACC CAG CTC ATA AAG GGT GTC GTC ATC GAC
AAG GAG      792
Ser Val Lys Asp Thr Gln Leu Ile Lys Gly Val Val Ile Asp Lys Glu
   210                215                220

GTC GTC CAC CCA GGC ATG CCG AAG AGG GTC GAG GGT GCT AAG
ATC GCC      840
Val Val His Pro Gly Met Pro Lys Arg Val Glu Gly Ala Lys Ile Ala
225                230                235                240

CTC ATC AAC GAG GCC CTC GAG GTC AAG GAG ACC GAG ACC GAC
GCC GAG      888
Leu Ile Asn Glu Ala Leu Glu Val Lys Glu Thr Glu Thr Asp Ala Glu
            245                250                255

ATC AGG ATC ACC AGC CCG GAG CAG CTC CAG GCC TTC CTT GAG
CAG GAG      936
Ile Arg Ile Thr Ser Pro Glu Gln Leu Gln Ala Phe Leu Glu Gln Glu
        260                265                270

GAG AAG ATG CTC AGG GAG ATG GTC GAC AAG ATC AAG GAG GTC
GGC GCG      984
Glu Lys Met Leu Arg Glu Met Val Asp Lys Ile Lys Glu Val Gly Ala
      275                280                285

AAT GTC GTC TTC GTC CAG AAG GGC ATT GAC GAC CTC GCC CAG
CAC TAC      1032
Asn Val Val Phe Val Gln Lys Gly Ile Asp Asp Leu Ala Gln His Tyr
   290                295                300

CTT GCC AAG TAC GGC ATA ATG GCC GTT AGA AGG GTC AAG AAG
AGC GAC      1080
Leu Ala Lys Tyr Gly Ile Met Ala Val Arg Arg Val Lys Lys Ser Asp

305                 310                 315                 320

ATG GAG AAG CTC GCC AAG GCC ACC GGC GCC AAG ATC GTC ACC
AAC GTC 1128
Met Glu Lys Leu Ala Lys Ala Thr Gly Ala Lys Ile Val Thr Asn Val
                325                 330                 335

CGC GAC CTC ACT CCG GAG GAC CTC GGT GAG GCC GAG CTC GTC
GAC CAG 1176
Arg Asp Leu Thr Pro Glu Asp Leu Gly Glu Ala Glu Leu Val Asp Gln
            340                 345                 350

AGG AAG GTC GCC GGC GAG AAC ATG ATC TTC GTC GAG GGC TGC
AAG AAC 1224
Arg Lys Val Ala Gly Glu Asn Met Ile Phe Val Glu Gly Cys Lys Asn
        355                 360                 365

CCG AAG GCC GTC ACA ATA CTC ATC AGG GGC GGC ACC GAG CAC
GTC GTT 1272
Pro Lys Ala Val Thr Ile Leu Ile Arg Gly Gly Thr Glu His Val Val
    370                 375                 380

GAT GAG GTC GAG AGG GCC CTT GAG GAC GCC GTC AAG GTC GTC
AAG GAC 1320
Asp Glu Val Glu Arg Ala Leu Glu Asp Ala Val Lys Val Val Lys Asp
385                 390                 395                 400

ATC GTC GAG GAC GGC AAG ATC GTC GCC GCC GGT GGT GCT CCG
GAG ATC 1368
Ile Val Glu Asp Gly Lys Ile Val Ala Ala Gly Gly Ala Pro Glu Ile
                405                 410                 415

GAG CTC GCC ATC AGG CTC GAC GAG TAC GCG AAG GAG GTC GGC
GGC AAG 1416
Glu Leu Ala Ile Arg Leu Asp Glu Tyr Ala Lys Glu Val Gly Gly Lys
            420                 425                 430

GAG CAG CTC GCC ATC GAG GCC TTT GCC GAG GCC CTC AAG GTC
ATC CCG 1464
Glu Gln Leu Ala Ile Glu Ala Phe Ala Glu Ala Leu Lys Val Ile Pro
        435                 440                 445

AGG ACC CTC GCC GAG AAC GCC GGT CTC GAC CCG ATC GAG ACC

CTC GTT     1512
Arg Thr Leu Ala Glu Asn Ala Gly Leu Asp Pro Ile Glu Thr Leu Val
   450                 455                 460

AAG GTC ATC GCC GCC CAC AAG GAG AAG GGA CCG ACC ATC GGT
GTT GAC     1560
Lys Val Ile Ala Ala His Lys Glu Lys Gly Pro Thr Ile Gly Val Asp
465                 470                 475                 480

GTC TTC GAG GGC GAG CCG GCC GAC ATG CTC GAG CGC GGC GTT
ATC GCC     1608
Val Phe Glu Gly Glu Pro Ala Asp Met Leu Glu Arg Gly Val Ile Ala
            485                 490                 495

CCG GTC AGG GTT CCG AAG CAG GCC ATC AAG AGC GCC AGC GAG
GCT GCC     1656
Pro Val Arg Val Pro Lys Gln Ala Ile Lys Ser Ala Ser Glu Ala Ala
         500                 505                 510

ATA ATG ATC CTC AGG ATC GAC GAC GTC ATC GCC GCC AGC AAG
CTC GAG     1704
Ile Met Ile Leu Arg Ile Asp Asp Val Ile Ala Ala Ser Lys Leu Glu
      515                 520                 525

AAG GAC AAG GAG GGC GGC AAG GGC GGT AGC GAG GAT TTC GGA
AGC GAC     1752
Lys Asp Lys Glu Gly Gly Lys Gly Gly Ser Glu Asp Phe Gly Ser Asp
   530                 535                 540

CTT    GAC    TGA    AGCCCTTTGA    TTTCTTTTCT    CTTCAAATTT
GTGTTCTTA          1800
Leu Asp  *
545

(2) INFORMATION FOR SEQ ID NO: 8:

(i) SEQUENCE CHARACTERISTICS:
(A) LENGTH: 547 amino acids
(B) TYPE: amino acid
(D) TOPOLOGY: linear

(ii) MOLECULE TYPE: protein

(xi) SEQUENCE DESCRIPTION: SEQ ID NO: 8:

Met Ala Gln Leu Ala Gly Gln Pro Val Val Ile Leu Pro Glu Gly Thr
1               5                   10                  15

Gln Arg Tyr Val Gly Arg Asp Ala Gln Arg Leu Asn Ile Leu Ala Ala
            20                  25                  30

Arg Ile Ile Ala Glu Thr Val Arg Thr Thr Leu Gly Pro Lys Gly Met
        35                  40                  45

Asp Lys Met Leu Val Asp Ser Leu Gly Asp Ile Val Ile Thr Asn Asp
    50                  55                  60

Gly Ala Thr Ile Leu Asp Glu Met Asp Ile Gln His Pro Ala Ala Lys
65                  70                  75                  80

Met Met Val Glu Val Ala Lys Thr Gln Asp Lys Glu Ala Gly Asp Gly
                85                  90                  95

Thr Thr Thr Ala Val Ala Ile Ala Gly Glu Leu Leu Arg Lys Ala Glu
            100                 105                 110

Glu Leu Leu Asp Gln Asn Ile His Pro Ser Ile Ile Ile Lys Gly Tyr
        115                 120                 125

Ala Leu Ala Ala Glu Lys Ala Gln Glu Ile Leu Asp Glu Ile Ala Lys
    130                 135                 140

Asp Val Asp Val Glu Asp Arg Glu Ile Leu Lys Lys Ala Ala Val Thr
145                 150                 155                 160

Ser Ile Thr Gly Lys Ala Ala Glu Glu Glu Arg Glu Tyr Leu Ala Glu
                165                 170                 175

Ile Ala Val Glu Ala Val Lys Gln Val Ala Glu Lys Val Gly Glu Thr
            180                 185                 190

Tyr Lys Val Asp Leu Asp Asn Ile Lys Phe Glu Lys Lys Glu Gly Gly
        195                 200                 205

Ser Val Lys Asp Thr Gln Leu Ile Lys Gly Val Val Ile Asp Lys Glu
    210                 215                 220

Val Val His Pro Gly Met Pro Lys Arg Val Glu Gly Ala Lys Ile Ala
225                 230                 235                 240

Leu Ile Asn Glu Ala Leu Glu Val Lys Glu Thr Glu Thr Asp Ala Glu
                245                 250                 255

Ile Arg Ile Thr Ser Pro Glu Gln Leu Gln Ala Phe Leu Glu Gln Glu
            260                 265                 270

Glu Lys Met Leu Arg Glu Met Val Asp Lys Ile Lys Glu Val Gly Ala
        275                 280                 285

Asn Val Val Phe Val Gln Lys Gly Ile Asp Asp Leu Ala Gln His Tyr
    290                 295                 300

Leu Ala Lys Tyr Gly Ile Met Ala Val Arg Arg Val Lys Lys Ser Asp
305                 310                 315                 320

Met Glu Lys Leu Ala Lys Ala Thr Gly Ala Lys Ile Val Thr Asn Val
                325                 330                 335

Arg Asp Leu Thr Pro Glu Asp Leu Gly Glu Ala Glu Leu Val Asp Gln
            340                 345                 350

Arg Lys Val Ala Gly Glu Asn Met Ile Phe Val Glu Gly Cys Lys Asn
        355                 360                 365

Pro Lys Ala Val Thr Ile Leu Ile Arg Gly Gly Thr Glu His Val Val
    370                 375                 380

Asp Glu Val Glu Arg Ala Leu Glu Asp Ala Val Lys Val Val Lys Asp
385                 390                 395                 400

Ile Val Glu Asp Gly Lys Ile Val Ala Ala Gly Gly Ala Pro Glu Ile
                405                 410                 415

Glu Leu Ala Ile Arg Leu Asp Glu Tyr Ala Lys Glu Val Gly Gly Lys
            420                 425                 430

Glu Gln Leu Ala Ile Glu Ala Phe Ala Glu Ala Leu Lys Val Ile Pro
        435                 440                 445

Arg Thr Leu Ala Glu Asn Ala Gly Leu Asp Pro Ile Glu Thr Leu Val

    450                 455                 460

Lys Val Ile Ala Ala His Lys Glu Lys Gly Pro Thr Ile Gly Val Asp
465                 470                 475                 480

Val Phe Glu Gly Glu Pro Ala Asp Met Leu Glu Arg Gly Val Ile Ala
                485                 490                 495

Pro Val Arg Val Pro Lys Gln Ala Ile Lys Ser Ala Ser Glu Ala Ala
            500                 505                 510

Ile Met Ile Leu Arg Ile Asp Asp Val Ile Ala Ala Ser Lys Leu Glu
        515                 520                 525

Lys Asp Lys Glu Gly Gly Lys Gly Gly Ser Glu Asp Phe Gly Ser Asp
    530                 535                 540

Leu Asp *
545

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 9:

AGGGGCCATG GCCCAGCTCG CAGGCCAGC 29

(2) INFORMATION FOR SEQ ID NO: 10:

(i) SEQUENCE CHARACTERISTICS:
(A) LENGTH: 28 base pairs
(B) TYPE: nucleic acid
(C) STRANDEDNESS: single

(D) TOPOLOGY: linear

(ii) MOLECULE TYPE: other nucleic acid

(xi) SEQUENCE DESCRIPTION: SEQ ID NO: 10:

AAAAGGGATC CAAGGTCATC AGTCAAGG 28

(2) INFORMATION FOR SEQ ID NO: 11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 11:

TGGTACCATG GCAAAGTATT CCGAACTCGA 30

(2) INFORMATION FOR SEQ ID NO: 12:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 12:

CGGATCCGAT ATCAGCTATG ACCTTTA 27

(2) INFORMATION FOR SEQ ID NO: 13:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 13:

GTGACCATGG GAAAGGCGCT GATGGTTCA 29

(2) INFORMATION FOR SEQ ID NO: 14:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 14:

CTAGGATCCA AGTCTCTGGA TTATGTACTG GA 32